(19) Europäisches Patentamt
European Patent Office
Office européen des brevets

(11) Veröffentlichungsnummer: **0 110 329**
**B1**

(12) # EUROPÄISCHE PATENTSCHRIFT

(45) Veröffentlichungstag der Patentschrift: **19.02.86**

(21) Anmeldenummer: **83111778.3**

(22) Anmeldetag: **24.11.83**

(51) Int. Cl.⁴: **C 07 C 69/533**, C 07 C 67/22, C 07 C 67/307, C 07 C 67/317, C 07 C 69/63, C 07 C 69/65

(54) **Verfahren zur Herstellung von alpha-Vinylpropionsäureestern.**

(30) Priorität: **30.11.82 DE 3244273**

(43) Veröffentlichungstag der Anmeldung: **13.06.84 Patentblatt 84/24**

(45) Bekanntmachung des Hinweises auf die Patenterteilung: **19.02.86 Patentblatt 86/8**

(84) Benannte Vertragsstaaten: **CH DE FR GB LI NL**

(56) Entgegenhaltungen:
**EP - A - 0 011 855**

**CHEMICAL ABSTRACTS, Band 94, Nr. 13, 30. März 1981, Seite 691, Nr. 102856s, Columbus, Ohio, USA**

(73) Patentinhaber: **BASF Aktiengesellschaft, Carl-Bosch-Strasse 38, D-6700 Ludwigshafen (DE)**

(72) Erfinder: **Ernst, Hansgeorg, Dr., Bruesseler Ring 38, D-6700 Ludwigshafen (DE)**
Erfinder: **Paust, Joachim, Dr., Ringstrasse 3, D-6708 Neuhofen (DE)**

EP 0 110 329 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine einfache Methode zur Herstellung von α-Vinylpropionsäureestern aus dem gut zugänglichen α-Vinylpropionitril.

α-Vinylpropionsäureester lassen sich durch Halogenanlagerung und Dehydrohalogenierung glatt in γ-Halogentiglinsäureester überführen, die in Form der entsprechenden Triarylphosphoniumsalze wertvolle $C_5$-Bausteine für die Terpenchemie darstellen.

Die Bildung von α-Vinylpropionsäureester ist bisher nur als Produkt einer Photoisomerisierung bekannt geworden [vgl. «Tetrahedron Letters», *1973* (49) 4889-92].

An der Herstellung dieser Ester besteht jedoch ein erhebliches präparatives Interesse, da sich aus ihnen, wie erwähnt, γ-Halogentiglinsäureester gewinnen lassen.

Es ist bekannt, γ-Halogentiglinsäureester aus α-Vinylmilchsäureester herzustellen. Letzere können jedoch wirtschaftlich nur gemäss der DE-OS Nr. 2852343 über die Umsetzung des toxischen Methylvinylketons mit Blausäure und anschliessende Überführung des Nitrils in eine Estergruppe hergestellt werden.

Es bestand daher die Aufgabe, aus einem grosstechnisch zugänglichen Ausgangsmaterial in einfacher Weise einen Weg zur Herstellung der γ-Halogentiglinester vorzuschlagen.

Diese Aufgabe wurde mit einer einfachen Synthese der α-Vinylpropionsäureester gelöst, die in die γ-Halogentiglinsäureester überführt werden können.

Es wurde nämlich überraschenderweise gefunden, dass man α-Vinylpropionitril in guter Ausbeute mit einer Lösung von Chlorwasserstoff in einem niedrigmolekularen Alkohol in die entsprechenden α-Vinylpropionsäureester überführen kann.

Der glatte Verlauf dieser «Pinner-Reaktion» ist überraschend, da wegen des stark salzsauren alkoholischen Mediums sowohl mit einer HCl-Anlagerung an die Doppelbindung als auch einer Isomerisierung mit Wanderung der Doppelbindung in Konjugation zur Estercarbonylgruppe zu rechnen war.

Die Umsetzung wird unter an sich für die Pinner-Reaktion bekannten Bedingungen durchgeführt. Man löst HCl-Gas in einem niedermolekularen Alkohol, vorzugsweise einem Alkohol mit 1 bis 4 C-Atomen und behandelt mit dieser Lösung α-Vinylpropionitril bei −15 bis +50° C, vorzugsweise bei 0 bis 20° C.

Die Konzentration an Chlorwasserstoff im Alkohol beträgt zweckmässig 15 Gew.-% bis zur Sättigung, und man wendet die Lösung des Chlorwasserstoffs in dem Alkohol in Mengen von 1,5 bis 10 mol (bezogen auf Chlorwasserstoff)/mol α-Vinylpropionitril an.

Als Alkohole kommen insbesondere Methanol und Ethanol in Betracht. n-Butanol und n-Propanol sind jedoch gleichermassen geeignet.

Im einzelnen geht man in der Regel so vor, dass man α-Vinylpropionitril zu der Lösung von Chlorwasserstoffgas im betreffenden Alkohol zutropft und mehrere Stunden bis 1 d ausreagieren lässt. Zur Hydrolyse wird dann auf Eis gegossen.

Der α-Vinylpropionester wird nach Alkalisierung des Gemisches, z. B. mit wässeriger Sodalösung, mit Ether oder Methylenchlorid extrahiert. Zur Abtrennung von Nebenprodukten ist eine Waschung der organischen Phase mit verdünnter Mineralsäure geeignet. Der α-Vinylpropionsäureester wird durch Eindampfen der organischen Phase und Destillation isoliert.

Die so erhältlichen α-Vinylpropionsäureester sind Öle, die unmittelbar weiter umgesetzt werden können.

Das Ausgangsmaterial α-Vinylpropionitril ist eine leicht zugängliche Chemikalie, die man beispielsweise durch Anlagerung von Blausäure an Butadien gemäss dem Verfahren der US-Patentschrift Nr. 3850973 erhält. Sie ist demnach auch ein Nebenprodukt der Adipodinitrilherstellung auf dem Wege der Anlagerung von Blausäure an Butadien.

Die erfindungsgemäss erhältlichen α-Vinylpropionsäureester lassen sich in einfacher Weise zum 2-Methyl-3,4-dihalogenbuttersäureester halogenieren.

Zur Herstellung der entsprechenden Chlorverbindungen gast man z. B. Chlorgas zweckmässig in äquimolarer Menge bis zu einem 10%igen Überschuss in eine Lösung von α-Vinylpropionsäureester in einem inerten Lösungsmittel, z. B. einem halogenierten Kohlenwasserstoff wie Tetrachlorkohlenstoff, ein oder man tropft zu dieser Lösung eine Lösung von Chlor in äquimolarer Menge oder geringem Überschuss ein.

Man erhält so die Dichlorester in hohen Ausbeuten.

In gleicher Weise werden die Dibromverbindungen durch Zutropfen einer äquimolaren Menge Brom, gelöst in einem halogenierten Kohlenwasserstoff, zu der Lösung des α-Vinylpropionsäureesters erhalten. Auch die Bromide werden in guter Ausbeute und solcher Reinheit erhalten, dass eine destillative Reinigung nicht erforderlich ist.

Die Dehydrohalogenierung der 2-Methyl-3,4-dihalogenbutsäureester erfolgt zweckmässig durch Behandeln der Dihalogenide in Methanol mit Natriummethylat oder in Ethanol mit Natriumethylat in 1- bis 1,1-fach molaren Mengen, bezogen auf das Dihalogenid, bei Temperaturen unterhalb 50° C, vorzugsweise bei Temperaturen zwischen 0 und 25° C.

Die Verwendung der so erhaltenen γ-Halogentiglinsäureester in Form der daraus mit Triarylphosphin erhältlichen Phosphoniumsalze der Formel

$$ROOC-\overset{\displaystyle CH_3}{\overset{|}{C}}=CH-CH_2-\overset{\oplus}{P}(Ar)_3X^{\ominus}$$

in der Ar einen Arylrest und X ein Halogenatom bedeutet, zur Herstellung von Terpenderivaten ist Gegenstand der deutschen Patentanmeldung P Nr. 3244772.6, auf die hiermit Bezug genommen wird.

*Beispiele:*

1. *α-Vinylpropionsäureethylester*

Zu 300 ml einer 43%igen Lösung von HCl-Gas in Ethanol wurden bei +5° C 40,5 g 2-Methyl-3-butennitril (75%ig), (≙ 0,375 mol) in 20 min zugetropft. Man rührte die Mischung 1 h bei 0 bis 5° C nach, und liess sie über Nacht bei Raumtemperatur stehen.

Dann wurde auf 250 g Eis gegossen, dreimal mit Methylenchlorid extrahiert, der Extrakt über $MgSO_4$ getrocknet und bei 105 torr/40° C eingeengt. Es verblieben 7,4 g Rückstand, der am Kugelrohr bei 135° C/100 torr destilliert wurde. Ausbeute: 1,56 g Destillat (GC-Gehalt an α-Vinylpropionsäureester 82% ≙ 1,28 g ≙ 3% d. Th.).

Die wässerige Phase wurde mit Soda alkalisch gemacht, dreimal mit Methylenchlorud extrahiert, über $MgSO_4$ getrocknet und bei 105 torr/40° C eingeengt. Der Rückstand wurde auf 100 ml 1 n HCl gegossen; dann wurde dreimal mit Ether extrahiert und der Extrakt über $MgSO_4$ getrocknet und eingeengt.

Destillation bei 98 mbar/73 bis 78° C lieferte 35,5 g Produkt mit einer GC-Reinheit von 93% (≙ 32,9 g) Gesamtausbeute: 68,7% d. Th.

2. *α-Vinylpropionsäuremethylester*

60,75 g 2-Methyl-3-butennitril (75%ig ≙ 0,563 mol) wurden bei 0° C in 300 ml einer bei 0° C gesättigten Lösung von HCl in absolutem Methanol getropft. Nach Erwärmen auf Raumtemperatur wurde die Mischung über Nacht nachgerührt.

Dann wurde auf Eis gegossen, mit gesättigter Sodalösung alkalisch gemacht, mit Methylenchlorid extrahiert und der Extrakt mit Kochsalzlösung gewaschen, über $MgSO_4$ getrocknet und eingeengt.

Die Destillation bei 54 bis 57° C/85 torr lieferte 33,9 g Produkt mit einem GC-Gehalt von 92,3% (≙ 48,8% d. Th.).

3. *2-Methyl-3,4-dichlorbuttersäuremethylester*

Zu einer Lösung von 9,92 g α-Vinylpropionsäuremethylester (92,3%ig ≙ 80 mmol) in 40 ml Tetrachlorkohlenstoff wurde bei 0° C eine Lösung von 5,7 g Chlor (80 mmol) in 60 ml Tetrachlorkohlenstoff in 1 h zugetropft und die Mischung 1 h bei Raumtemperatur nachgerührt. Nach Abziehen des Lösungsmittels am Rotationsverdampfer verblieben 14,7 g klare Flüssigkeit als Rückstand. Gehalt Dichlorid: 96% (≙ 95,4% d. Th.).

Das Rohprodukt kann direkt weiter umgesetzt werden.

4. *2-Methyl-3,4-dichlorbuttersäureethylester*

8,9 g (94,8%ig ≙ 65,9 mmol) α-Vinylpropionsäureethylester wurden in 120 ml Tetrachlorkohlenstoff gelöst. Bei 0 bis +5° C wurden innerhalb 1 h 5 g Chlor (70 mmol) eingegast. Man liess das Gemisch auf Raumtemperatur kommen und engte am Rotationsverdampfer ein.

Ausbeute: 13,6 g einer klaren Flüssigkeit. Gehalt an 2-Methyl-3,4-dichlorbuttersäureethylester gaschromatographisch bestimmt: 93,7%ig ≙ 12,74 g (97% d. Th.). Das Rohprodukt wurde direkt weiterverarbeitet.

5. *2-Methyl-3,4-dibrombuttersäuremehylester*

5 g α-Vinylpropionsäuremethylester (92,3%ig ≙ 40,5 mmol) wurden in 30 ml Tetrachlorkohlenstoff gelöst. Bei 0° C wurden 6,6 g Brom (41,25 mmol) in 20 ml Tetrachlorkohlenstoff innerhalb von 1 h zugegeben. Man rührte 1 h bei 0° C nach und liess die Mischung über Nacht bei Raumtemperatur stehen.

Nach dem Eindampfen am Rotationsverdampfer wurden 8,9 g schwach gelbes Öl erhalten.

Gaschromatographisch bestimmter Gehalt an Dibromid: 94% (≙ 75,4% d. Th.).

6. *γ-Bromtiglinsäuremethylester*

6,68 g 2-Methyl-3,4-dibrombuttersäuremethylester (94%ig ≙ 22,9 mmol) wurden in 10 ml absolutem Methanol gelöst. Im Laufe von 10 min wurden 4,1 g 30%iger methanolischer Natrium-Methylatlösung zugetropft, wobei die Temperatur durch Kühlung auf +20° C gehalten wurde. Dann wurde 1 h bei Raumtemperatur nachgerührt, auf 2 m HCl gegossen, mit Methylenchlorid extrahiert, der Extrakt mit Sodalösung gewaschen, über $MgSO_4$ getrocknet und eingeengt.

Rückstand: 4,3 g mit gaschromatographisch bestimmtem Gehalt an 87% (= 85% d. Th.) γ-Bromtiglinester. Das Produkt wurde durch Destillation am Kugelrohr bei 25 torr/120 bis 140° C gereinigt. Ausbeute: 3,1 g.

7. *γ-Chlortiglinsäuremethylester*

1,84 g 2-Methyl-3,4-dichlorbuttersäureethylester (95,4%ig ≙ 9,49 mmol) wurden in 10 ml Methanol gelöst, und bei +15 bis 20° C wurden 1,7 g einer 30%igen Lösung von Natrium-Methylat in absolutem Methanol zugetropft. Man rührte noch 1,5 h bei Raumtemperatur nach, goss auf 2 n HCl und extrahierte mit Methylenchlorid. Nach Waschen des Extrakts mit Hydrogencarbonatlösung, Trocknen über $MgSO_4$ und Einengen wurden 1,3 g γ-Chlortiglinester mit einem Gehalt von 94% isoliert (≙ 79,2% d. Th.).

8. *γ-Chlortiglinsäureethylester*

Zu einer Lösung von 12,87 g 2-Methyl-3,4-dichlorbuttersäureethylester (93,7%ig ≙ 60,6 mmol) in 10 ml absolutem Ethanol tropfte man bei 0 bis +5° C eine Lösung von 1,55 g Natrium (67,4 mmol) in 40 ml absolutem Ethanol im Laufe von 15 min zu. Nach weiterem einstündigem Rühren bei Raumtemperatur wurde auf 2 m HCl gegossen, dreimal mit Methylenchlorid extrahiert, der Extrakt mit Natriumbicarbonat-Lösung gewaschen, über Magnesiumsulfat getrocknet und am Rotationsverdampfer eingeengt.

Ausbeute: 10,0 g klare Flüssigkeit mit einem Gehalt an Chlortiglinester von 93,8% ≙ 95,3% d. Th.

Nach Destillation am Kugelrohr bei 33 torr bis 150° C erhielt man 9,0 g γ-Chlortiglinsäureethylester als Gemisch aus E/Z-Isomeren im Verhältnis E:Z = 70:30 (nach NMR).

**Patentansprüche**

1. Verfahren zur Herstellung von α-Vinylpropionsäureestern der Formel

$$\begin{array}{c} \quad\; CH_3 \\ \quad\; | \\ CH_2=CH-CH-COOR \end{array}$$

dadurch gekennzeichnet, dass man α-Vinylpropionitril mit einer Lösung von Chlorwasserstoff in einem Alkohol ROH bei −15 bis +50° C umsetzt, wobei R einen niedermolekularen Rest bedeutet.

2. Vrfahren gemäss Anspruch 1, dadurch gekennzeichnet, dass man die Umsetzung bei 0 bis 20° C durchführt.

3. Verfahren gemäss Anspruch 1, dadurch gekennzeichnet, dass man mit einem Alkohol mit 1 bis 4 Kohlenstoffatomen umsetzt.

4. Verwendung der gemäss Anspruch 1 α-Vinylpropionsäureester zur Herstellung von γ-Halogentiglinsäureester durch Halogenaddition an die Vinylgruppe und Dehydrohalogenierung.

**Claims**

1. Process for the preparation of an α-Vinylpropionate of the formula

$$\begin{array}{c} \quad\; CH_3 \\ \quad\; | \\ CH_2=CH-CH-COOR \end{array}$$

wherein α-vinylpropionitrile is reacted with a solution of hydrogen chloride in an alcohol ROH at from −15 to +50° C, R being low molecular weight radical.

2. A process as claimed in Claim 1, wherein the reaction is carried out at from 0 to 20° C.

3. A process as claimed in Claim 1, wherein the reaction is carried out using an alcohol of 1 to 4 carbon atoms.

4. The use of an α-vinylpropionate, obtainable as claimed in Claim 1, for the preparation of an γ-halotiglate by the addition of halogen to the vinyl group, and dehydrohalogenation.

**Revendications**

1. Procédé pour la préparation d'esters d'acide α-vinylpropionique de formule

$$\begin{array}{c} \quad\; CH_3 \\ \quad\; | \\ CH_2=CH-CH-COOR \end{array}$$

caractérisé en ce qu'on fait réagir l'α-vinylpropionitrile, à une température de −15 à +50° C, avec une solution d'acide chlorhydrique dans un alcool ROH, R représentant un radical de faible poids moléculaire.

2. Procédé selon la revendication 1, caractérisé en ce qu'on conduit la réaction à une température de 0 à 20° C.

3. Procédé selon la revendication 1, caractérisé en ce qu'on mène la réaction avec un alcool à 1 à 4 atomes de carbone.

4. Utilisation des esters d'acide α-vinylpropionique obtenus suivant la revendication 1, pour la préparation d'esters d'acides γ-halogénotigliques par addition d'halogène sur le groupe vinyle et déshydrohalogénation.